(19) Europäisches Patentamt
European Patent Office
Office européen des brevets

(11) **EP 1 896 173 B1**

(12) **EUROPEAN PATENT SPECIFICATION**

(45) Date of publication and mention
of the grant of the patent:
**05.10.2011 Bulletin 2011/40**

(51) Int Cl.:
***B01J 21/06*** (2006.01)

(21) Application number: **06733431.8**

(22) Date of filing: **24.05.2006**

(86) International application number:
**PCT/SE2006/000607**

(87) International publication number:
**WO 2006/126946 (30.11.2006 Gazette 2006/48)**

(54) **METHOD FOR PHOTOCATALVTIC OXIDATION OF ORGANIC SUBSTANCES IN AIR**

VERFAHREN ZUR PHOTOKATALYTISCHEN OXIDATION VON ORGANISCHEN SUBSTANZEN IN
LUFT

PROCEDE D'OXYDATION PHOTOCATALYTIQUE DE SUBSTANCES ORGANIQUES PRESENTES
DANS L'AIR

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **24.05.2005 SE 0501169**

(43) Date of publication of application:
**12.03.2008 Bulletin 2008/11**

(73) Proprietor: **Totalförsvarets Forskningsinstitut
164 90 Stockholm (SE)**

(72) Inventor: **ÖSTERLUND, Lars
S-906 42 Umeå (SE)**

(74) Representative: **Hedefält, Dag et al
Försvarets Materielverk
Patentenheten
115 88 Stockholm (SE)**

(56) References cited:
WO-A-02/06159          US-A1- 2002 029 955
US-A1- 2003 150 707   US-B1- 6 342 128

- **LINSEBIGLER A.L. ET AL.: 'Photocatalysis on
  TiO2 surfaces: Principles, mechanisms and
  selected results' CHEM. REV. vol. 95, 1995, pages
  735 - 758, XP003000356**
- **DEMEESTERE K. ET AL.: 'Adsorption as initial
  step in the photocatalysis degradation of
  gaseous VOC by TiO2/UV' 8TH FECS
  CONFERENCE 2002, ESPR-RENVIRON. SCI. &
  POLLUT. RES. no. 3, pages 144 - 145,
  XP003000357**

**Description**

The invention relates to a method for photocatalytic oxidation of organic substances in air on a photocatalytic surface of semiconductive metal oxide, air containing the organic substances being caused to flow over the photocatalytic surface and the surface being irradiated with activating light. The photocatalytic material absorbs light (photons) and generates excitons (electron-hole pairs) which after diffusion to the surface either directly or indirectly by generation of radicals adhere to molecules bound to the surface and thus initiate a (catalytic) chemical reaction in the interface of the 2-phase system.

Heterogeneous phase photocatalytic degradation of the above type is previously known and described, for instance, in Fujishima, A.; Hashimoto, K.; Watanabe, T. TiO2 Photocatalysis. Fundamentals and Applications BKC, Inc.: Tokyo 1999; Ollis, D.F., Al-Ekabi, H., Photocatalytic Purification and Treatment of Water and Air, Eds., Elsevier: Amsterdam, 1993, Fox, M.A. Dulay, M.T. Chem. Rev. 1993, 93, 341; Mills, A.; Le Hunte, S. J. Photochem. Photobiol. A 1997, 108, 1.

In WO 02/06159 A, Kemitt et al, it is disclosed methods to prepare titania based coatings on various substrates and applications of these materials to photooxidation of gaseous organic pollutants, in particular photothermal degradation of ethane gas in humidified air over a platinised titania based coating on woven glass cloth in a closed batch reactor. This document does not disclose a preferred number of monolayers of water molecules provided on a photocatalytic surface of a semiconductive metal oxide.

In US 2003/15707 A1, Carmignani et al it is disclosed methods to device an apparatus for effective photocatalytic purification and disinfection of liquid fluids by means of passing the fluid through a semitransparent pack bed through a tortuous path. This document does not disclose a method to obtain a photocatalytic oxidation by means of providing the photocatalytic surface with a preferred number of monolayers of water molecules.

A problem in photocatalytic oxidation of organic substances in air is that the efficiency (that is the number of molecules oxidised per incident photon) is low, and that the catalyst tends to be deactivated and lose efficiency (conversion of molecules per unit of time) after a certain time of operation. In deactivation, organic or inorganic degradation products are firmly bound to the surface. In outdoor applications, the photocatalyst can often be regenerated by rainwater flushing the surface clean, but in other applications the need for recurrent activation and regeneration measures will be a problem, for instance when the photocatalyst is incorporated into a reactor through which air circulates.

The object of the present invention is to achieve a long-term degradation process of organic substances on a photocatalytic surface without the catalyst being deactivated, that is without the degree of conversion being reduced. Another object is to optimise the photocatalytic process. A further object is to provide a convenient device for cleaning of air.

This is achieved by a method and a device as defined in the claims.

According to the invention, the temperature of the photocatalytic surface ($T_{cat}$) are regulated so that the combination of $RH_{air}$ and $T_{cat}$ is caused to fall within predetermined acceptable combinations of $RH_{air}$ and $T_{cat}$ to establish and maintain 0.2-8 monolayers of water molecules on the photocatalytic surface.

The establishment of a water layer on the surface of the photocatalyst is a condition for the photocatalyst to avoid being deactivated. The establishment of a thin (0.2-8 ML) water layer besides optimises the photocatalytic degradation process.

Titanium dioxide ($TiO_2$) is the photocatalytic active material, but also other similar semiconductive metal oxides can be used, including doped metal oxides, binary and tertiary oxides. The photocatalyst can be in the form of a coating on a substrate of another material, in pure crystalline form or powder form containing mixtures of different phases (polymorphics) and other metal oxides.

The organic substances which can be oxidised with the photocatalyst according to the invention are all compounds containing carbon (C), hydrogen (H) and optionally oxygen (O) or nitrogen (N). They may also contain electronegative elements such as phosphorus (P), chlorine (CI), fluorine (F) and sulphur (S).

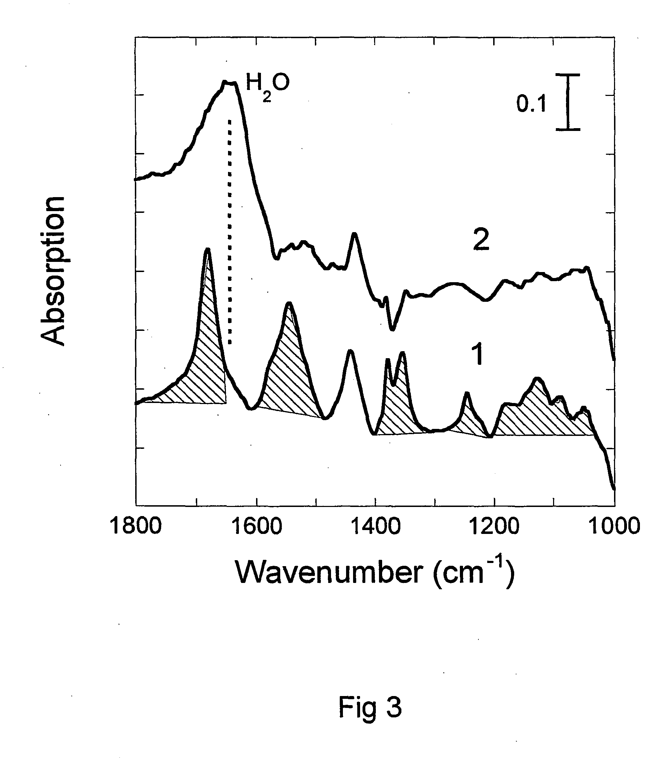

The inventive method is based on avoiding the reactions on the photocatalyst which cause generation of stable surface-bound compounds, which inactivate the photocatalyst. By regulating the humidity in the reaction environment and the temperature of the catalyst so that 0.2-8 monolayers of water are established on the catalyst surface, degradation products are prevented to form, which are coordinated with surface atoms and form strong surface bonds. In the case of $TiO_2$, such surface atoms can be undercoordinated metal atoms, such as 5-fold coordinated Ti atoms, which are dependent on the structure of the surface and have an effective charge which differs from bulk $TiO_2$ by being less positively charged. Examples of such stable degradation products are bridge-bonded format (HCOO), carbonate ($CO_3$), phosphate ($PO_4$), sulphate ($SO_4$) or halides (for instance M-F; if F is an integral element). These are general products which are formed on the surface of the photocatalyst in total oxidation of $C_xH_yA_zO_vB_w$ (A, B=CI, S, F or P) compounds, and which strongly bond to the undercoordinated metal atoms on the surface of the photocatalyst, as shown in Example 3 and Fig. 3 below. By regulating $RH_{air}$ in the reaction environment in which the photocatalyst is positioned and controlling the temperature of the photocatalyst ($T_{cat}$) to predetermined acceptable combinations of $RH_{air}$ and $T_{cat}$, it is possible to build or maintain the thin layer of water on the surface. $RH_{air}$ can be regulated by an exact amount of water being supplied to the air or by the air being dried and/or the temperature of the air being adjusted. The thin water layer

(i) prevents dehydroxylation of the metal oxide surface,
(ii) prevents $C_xH_yO_z$, $CO_x$, $PO_x$, $SO_x$ and halogen compounds from binding strongly to the catalyst surface,
(iii) allows excitons (electron-hole pairs) from the photocatalyst and radicals formed on the same to effectively reach the reactants without their being recombined.

The thin water layer should be between 0.2 and 8 monolayers (ML). Especially preferred is between 0.3 and 6 ML, in particular between 0.4 and 3 ML for optimum effect. A monolayer corresponds to $1.15 \cdot 10^{19}$ $H_2O$ molecules per $m^2$ on $TiO_2$ and is the number of $H_2O$ molecules that are necessary to cover the entire surface. This amount of water also includes water which is split into OH groups and which regenerates M-OH surface compounds and thus prevents dehydroxylation and the subsequent reduction of the surface (and, for instance, exposure of undercoordinated metal atoms).

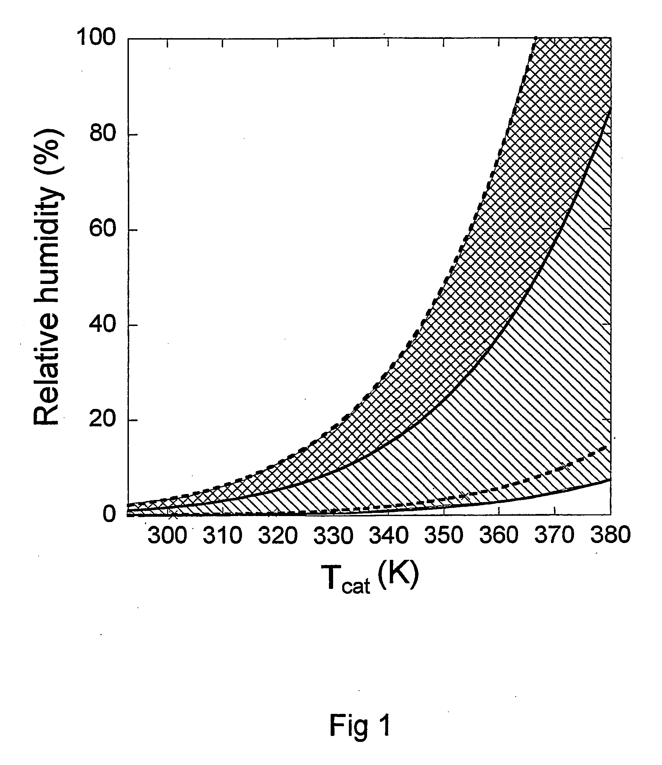

Since the amount of water on the surface is affected by the temperature of the photocatalyst surface, the humidity can be adjusted to the temperature of the photocatalyst, or vice versa. Based on the thermodynamic parameters of the system $H_2O$/metal oxide, and the sublimation energy of $H_2O$, it is possible to calculate suitable combinations of $RH_{air}$ and $T_{cat}$ which correspond to the range which gives a desired thin water layer on the catalyst and an optimised photocatalytic activity. Example 1 below shows how such a calculation can be made for the system $H_2O/TiO_2$ and a diagram of acceptable combinations of $RH_{air}$ and $T_{cat}$ is constructed. For instance, for a $TiO_2$ catalyst, the relative humidity ($RH_{air}$) is to be adjusted from 0.01 % to 5%, preferably between 0.05% and 4%, and most advantageously between 0.1 % and 2%, when the catalyst ($T_{cat}$) operates at room temperature and has ambient air with a temperature of 298 K and an air pressure of 1013 mbar. At a higher catalyst temperature, the RH should be between higher values. When the temperature of the catalyst surface, $T_{cat}$, is 360 K, the RH should be, for instance, between 1% and 60%, preferably between 2% and 50%, and most advantageously between 3% and 40% under otherwise identical conditions. It is noted that when the catalyst operates at room temperature, it is normally necessary to dry the air to optimise the photocatalytic activity, whereas at an increased catalyst temperature, it may be necessary to humidify the air. The temperature of the catalyst surface, $T_{cat}$, and the temperature of the ambient air can thus be different. The most favourable operating conditions are achieved when the catalyst surface has an increased temperature relative to the ambient air. When air at room temperature is treated with a catalyst with an increased $T_{cat}$, the catalyst functions with high activity in a wide RH range, as shown in the following with reference to Fig. 1. In addition, the velocity of the thermally conditioned reaction steps increases.

The method according to the invention makes it possible to optimise the photocatalytic degradation of organic compounds $C_xH_yA_zO_vB_w$ (A, B=Cl, S, F or P) at an increased temperature of the catalyst according to the following principle. In the photocatalytic oxidation, intermediate products are primarily formed, which react further by thermally activated reaction steps. An increased catalyst temperature significantly increases the velocity of these thermally activated reaction steps. The temperature of the photocatalyst is, however, not allowed to exceed a temperature which makes impossible the maintenance of a concentration of water on the photocatalyst surface of at least 0.2 ML. Preferably the water layer should be at least 0.3 ML and most advantageously at least 0.5 ML. This means that an upper temperature limit exists which is dependent on the total pressure over the surface of the photocatalyst. In particular, the photocatalyst is not allowed to be warmer than 440 K at an air pressure of 1 atm, preferably not higher than 400 K and most advantageously not higher than 390 K, to achieve the synergistic effect of thermal degradation and optimal photocatalytic degradation by a maintained thin water layer in equilibrium with vapour phase.

The catalyst is irradiated with light having an energy which exceeds the optical bandgap energy of the photocatalyst. For $TiO_2$, this is 3.2 eV or 388 nm (antase modification) and respectively 3.0 eV or 414 nm (rutile modification). A photocatalyst of $TiO_2$ can be illuminated with UV lamps which emit wavelengths in the range 300-400 nm (UVA). A suitable irradiance (illuminated effect per area unit) is from 5 $W/m^2$ to 500 $W/m^2$, especially between 10 $W/m^2$ and 300 $W/m^2$. These lamps can be so-called BLB UV lamps (black-light bulbs), other gas discharge lamps or light-emitting diodes which emit UVA light as stated above. It is to be noted that heat from the UVA lamp with the correct geometric design can also be used to heat the photocatalyst in order to optimise the photocatalytic activity. The photocatalytic layer can in such a case be arranged as a thin film on the light source and $T_{cat}$ can be regulated by regulating the effect of the light source or electric supplementary heating of the casing of the light source. The film must be thinner than the penetration depth of the activating light. The thickness of a $TiO_2$ coating on the light source should be, for instance, less than 2 micrometer, preferably less than 1 micrometer.

The method according to the invention can be applied in various contexts where air is to be cleaned of organic substances. The photocatalyst can be installed in all closed spaces with controllable circulation of air. This includes existing ventilation systems; separate air cleaners; mobile air cleaning plants, in all types of vehicles, such as cars, trucks, airplanes and ships where it is possible to control RH and temperature and airflows (velocity and type of flow). Reading of the RH can take place using existing commercial RH meters. The RH can be controlled by temperature where supply of heat can take place by heat exchange, and/or spiral heating, or by humidifying the air with existing air humidifiers which can be injection systems, or water bath. In many cases, the RH can be too high and then the air must be dried before passing over the photocatalyst. This can easily be performed using a siccative, for instance hygroscopic materials such as silica gel, activated clay and aluminium silicate, which have a large inner area.

When the concentration of organic compounds in the air varies over time, an adsorption filter can be used to equalise the load on the photocatalyst. The filter can be dimensioned to only adsorb a fraction of the organic substances, or only a fraction of the air is passed through the filter. In periods of a low concentration of organic substances in the air, the filter can be desorbed at a controlled rate and the temporarily adsorbed substances can be supplied to the photocatalyst.

The air can also be filtered through a particle filter before it is contacted with the catalyst to prevent the photocatalytic surface from being contaminated with dust and other solid particles.

The control of the catalyst temperature can be coordinated with the control of the RH, and vice versa. This is achieved by $RH_{air}$ and $T_{cat}$ being controlled in an integrated regulating system. Control and adjustment of RH and temperature can also take place in several steps before the air is exposed over the photocatalyst. The airflow rate can be controlled by fans, by feedback regulation towards a suitably placed flow meter, and throttle and expansion valves in suitable positions.

The method according to the invention can advantageously be combined with different coating methods and techniques, including multilayer coatings to achieve a high photocatalytic activity. For instance, antistatic layers between the photocatalyst and the substrate can be used with polarisation of the layers (by electric contacting and supply of current) in order to concentrate particles on the photocatalyst and increase the dwell time of the same and, thus, the efficiency of the absolute photocatalytic purification. Antireflective layers can be used for refractive index matching and to control the absorption of the activating light.

Electrochromic layers can be used, for instance, to absorb light and thus increase the temperature of the layer and the absorbed heat can be transferred by heat conduction to the photocatalytic layer. The photocatalytic surface can be arranged on a light-absorbing layer, which is substantially transparent to UV light but absorbs visible and infrared light. Electrochromic materials are examples of such light-absorbing layers. Light, such as sunlight, which falls on the light-absorbing layer, is absorbed in the same and thus increases the temperature of the layer. UV light that is not absorbed continues through the layer so as to reach the photocatalytic layer. By arranging a photocatalytic layer on the light-absorbing layer, also an increased temperature of the photocatalytic surface is obtained, if the photocatalytic layer is sufficiently thin. The thickness of the photocatalytic layer controls the temperature $T_{cat}$ of the surface of the photocatalyst and can be used for additional optimisation. The temperature gradient in the photocatalytic layer results in a thin layer giving a higher temperature of the photocatalytic surface than a thick layer. Microstructures such as particle size and porosity also affect heat conduction and temperature profile in the photocatalytic layer, and, in conjunction with the photocatalytic function, also these parameters can be optimised. The photocatalytic layer must, however, always be thinner than the penetration depth of light with energy that exceeds the optical bandgap energy of the photocatalyst for the light to reach the surface where the photocatalytic reactions are to be initiated. For $TiO_2$, the layer should be less than 1 micrometer, preferably less than 0.5 micrometer, and most advantageously less than 0.2 micrometer. A photocatalytic device constructed according to this principle, which is illuminated with, for instance, sunlight on the light-absorbing layer, thus acts at a higher $T_{cat}$, which on the one hand gives an increased reaction rate of photocatalytic degradation and, on the other hand, allows a higher RH for optimal effect than if the photocatalyst acted at room temperature. Conversely, the same principle can be applied by illumination from the opposite direction, that is incident light illuminates a photocatalytic layer so that the light reaches a subjacent light-absorbing material. In this case, the photocatalyst should be sufficiently transparent in a wavelength range which is greater than the optical bandgap energy of the photocatalyst for light radiation to reach the light-absorbing layer. In the same way as mentioned above, also the thickness of the photocatalytic layer is important to how warm the surface will be by heat conduction from the light-absorbing layer.

Examples of photocatalytic reactor systems can be photocatalytic window constructions, air cleaners, membrane structures, waveguides (optical fibre constructions) etc. Photocatalytic materials are available in the form of powder and coatings on glass, paper, polymer films, glazed tiles etc, which allows different geometries of reactor constructions depending on application.

The invention will now be described by way of examples and embodiments with reference to the accompanying Figures.

Fig. 1 is a graphic display of acceptable combinations of $RH_{air}$ and $T_{cat}$ according to the invention for a $TiO_2$ catalyst which is supplied with an airflow having a temperature of 25°C and a pressure of 1013 mbar.

Fig. 2 shows graphs of conversion of propane in air as a function of $T_{cat}$ for different levels of $RH_{air}$ for a $TiO_2$ catalyst.

Fig. 3 shows FT-IR spectra after photocatalytic degradation of a halogenated carbon compound on a $TiO_2$ catalyst in dry air and $RH_{air}$ according to the invention.

Fig. 4 shows schematically a simple application of the invention for cleaning of air with active regulation of $T_{cat}$ and measuring of $RH_{air}$.

Fig. 5 shows schematically an embodiment for installation in an air-conveying system.

Fig. 6 is a detailed view of an embodiment of a tubular photocatalytic reactor according to Fig. 5.

Fig. 7 shows schematically an application of the invention in an air-cleaning system with integrated regulation of $T_{cat}$ and $RH_{air}$.

Example 1

This Example shows how, for the system $H_2O/TiO_2$, suitable combinations of $RH_{air}$ and $T_{cat}$ are calculated, which result in a desired thin water layer on a $TiO_2$ catalyst.

Given that the adsorption energy of the water, $E_a$, on the photocatalyst is known or can be measured (on $TiO_2$ it is known and about 12 kcal/mol), the surface concentration of the water in equilibrium with a vapour phase atmosphere containing water vapour (given by $RH_{air}$) can be estimated. The following designations are used:

| | |
|---|---|
| $k_d$ | = Rate at which $H_2O$ molecules leave the catalyst surface |
| $v_o$ | = preexponential factor $\approx 10^{13} S^{-1}$ |
| $v$ | = Number of $H_2O$ molecules hitting the catalyst surface per unit of time |
| $k_a$ | = Rate at which $H_2O$ molecules hit the catalyst surface |
| $S_0$ | = Probability that an $H_2O$ molecule sticks to the catalyst surface |
| $E_d$ | = Desorption energy of $H_2O$ molecules from the catalyst surface |
| $N_{H2O}$ | = Number of $H_2O$ molecules per $m^2$ on the photocatalyst |
| $P_{H2O}$ | = Partial pressure of $H_2O$ in the ambient gas (air) |
| M | = Molar mass of $H_2O$ = 18 g/mol |
| R | = General gas constant = 8314 J/mol K |
| T | = Temperature of ambient gas (air) |
| $T_{cat}$ | = Temperature of photocatalyst |

It can be assumed that $k_a = s_0*v$ for $H_2O$, so that in equilibrium, the number of molecules leaving (desorbing) and adsorbing the catalyst surface per unit of time must be the same, viz.

$$(1) \qquad k_a = k_d$$

It can also be assumed that the following estimates apply to water ($E_d = E_a$):

$$(2) \qquad k_d = v_0 * exp(-E_d/T_{cat}) * N_{H2O}$$

and

$$(3) \qquad v = P_{H2O} * s_0 / \sqrt{(2\pi MRT)}$$

By combining equations 1-3, and the condition that $0.4 < \theta_{H2O} < 3$ ML, where 1 ML is defined as $1.15*10^{19}$ $H_2O$ molecules/$m^2$, the desired parameter space ($P_{H2O}$, $T_{cat}$) is obtained, which gives the optimal photocatalytic conditions (where it is assumed that $S_0 = 1$ in these calculations). Correspondingly, for instance the ambient temperature can also be adjusted to a desired value.

Fig. 1 shows the graph of $RH_{air}$ as a function of $T_{cat}$ when the photocatalyst is kept in an ambient atmosphere at 298 K and 1013 bar when the saturation pressure of $H_2O$ is 3172 Pa. The dashed area with the solid lines indicates the upper and lower limit of the theoretically optimised $RH_{air}$ vs the $T_{cat}$ graph calculated as stated above with the given condition that at 380 K the equilibrium coverage of the photocatalyst is 1 ML and $10<E_d<12$ kcal/mol where the lower limit indicates the sublimation energy of water. The checkered area with the dashed lines indicates the upper and lower limit of the theoretically optimised $RH_{air}$ vs the $T_{cat}$ graph calculated as stated above with the given condition that at 380 K the equilibrium coverage of the photocatalyst is 2 ML and $10<E_d<12$ kcal/mol. In the latter case, the upper limit of $T_{cat}$ is given by $T_{cat}$=367 K in order to maintain 2 ML on the surface ($RH_{air}$ = 100%). It is evident from the Figure that when air of room temperature is treated with a catalyst which has an increased $T_{cat}$, the acceptable range of $RH_{air}$ increases significantly. This condition also applies in case of moderate changes of the temperature of the air, which means that it is possible to tolerate a certain heating of the air, and thus a reduction of $RH_{air}$, in the contact with the catalyst. The $RH_{air}$ of the input air can be adjusted to a level that can be reduced during the passage of the catalyst, and still be at a level which is acceptable in combination with a prevailing increased $T_{cat}$. Furthermore, for an increased $T_{cat}$, acceptable $RH_{air}$ ranges for optimal photocatalytic activity according to Fig. 1 allow values of $RH_{air}$ which are normally to be found in indoor environment ($RH_{air}$ = 30-70%), and therefore the need for drying the air decreases.

Example 2

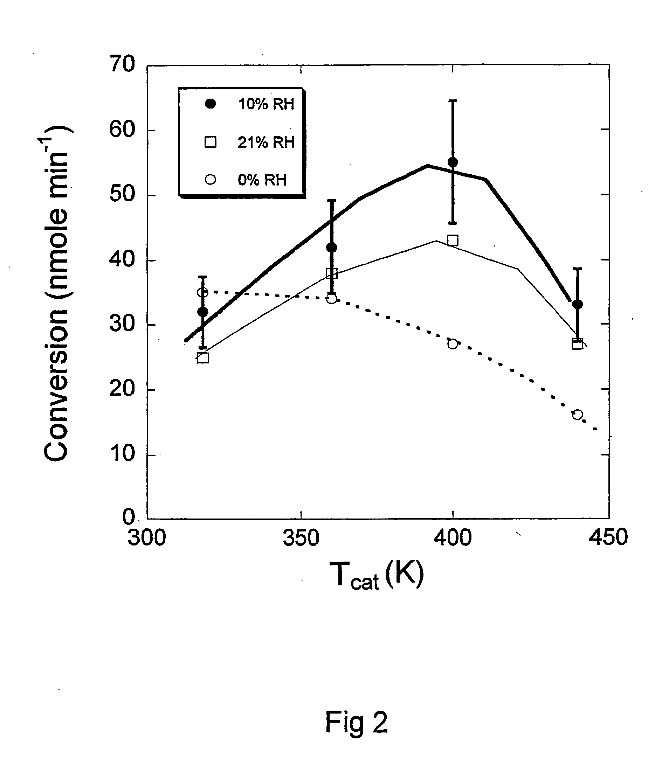

The conversion of propane in a gas flow flowing over a $TiO_2$ catalyst was measured as a function of $T_{cat}$ at $RH_{air}$ = 0.10 and 21%. The flow of gas (30 ml/min) consisted of 500 ppm propane gas in synthetic air and had a temperature of 318 K and a pressure of 1013 mbar. The gas was humidified by injection of water through a capillary in connection with a pressurised container. $TiO_2$ in antase modification was illuminated with a 150 W Xe lamp. The conversion of the reactants over the photocatalyst was registered by a quadrupole mass spectrometer. The result is shown in Fig. 2. As is evident from the Figure, the conversion decreased significantly as a function of an increasing $T_{cat}$ for $RH_{air}$ = 0% when $RH_{air}$ was outside the acceptable range marked in Fig. 1. An optimisation of the activity was possible by regulating $RH_{air}$.

Example 3

This Example elucidates that the occurrence of stable surface-bound compounds can be avoided by adjusting $RH_{air}$ to a level which is suited for the current catalyst temperature, $T_{cat}$. FT-IR spectra were measured after photocatalytic degradation of diisopropyl-fluorophosphate (DFP), $C_6H_{14}FO_3P$, on $TiO_2$ nanoparticles (<d>=33 nm; specific surface about 50 $m^2$/g). A high content of DFP (11 $\mu$g/min) was evaporated in air and was made to flow over a bed of $TiO_2$ metal oxide particles for 20 min and after that the bed was illuminated with simulated sunlight (AM 1.5) for 60 min. The photocatalyst had a temperature of 310 K. The result is shown in Fig. 3. The lower spectrum (1) shows the result in dry air and the upper (2) with a small amount of water added to the air for adjusting $RH_{air}$ to about 9%. The results unambiguously show that smaller amounts of surface-bound format, carbonate and phosphate compounds are bound to the photocatalyst surface in an environment of controlled relative humidity of the air, which is a condition to prevent deactivation and reduced efficiency. When optimising $RH_{air}$ according to the invention, the concentration of surface-bound compounds (the dashed IR absorption peaks in graph 1) is reduced, while at the same time water is adsorbed on the surface (dashed vertical line). Photoelectron spectroscopy (XPS) showed that also the concentration of inorganic F (Ti-F compounds) that was built up in the dry case was below detection level after reaction in controlled relative humidity.

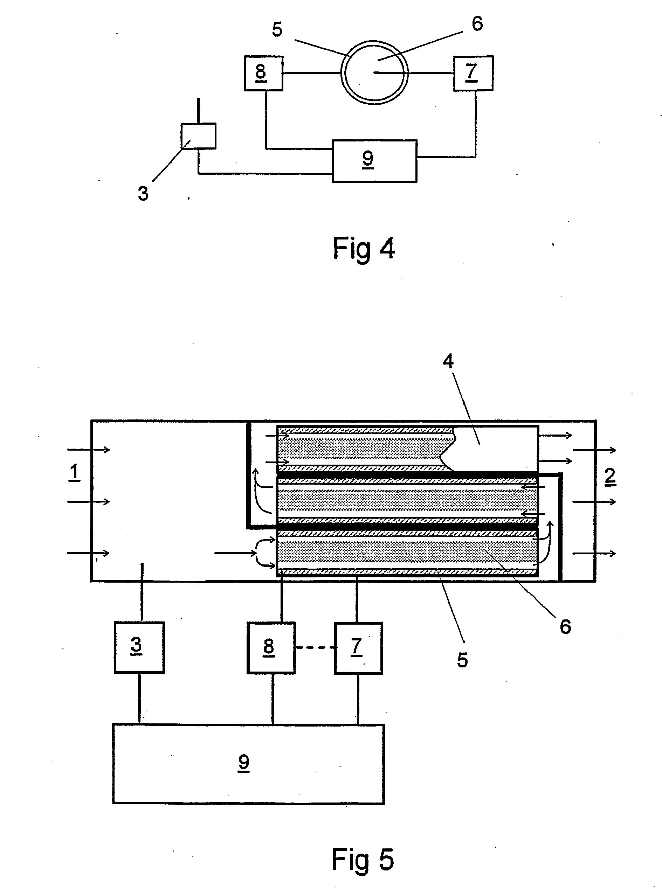

Air cleaning systems will be described in the following with reference to Figs 4-7.

Equivalent components in the Figures have been given the same reference numerals.

In many cases, it may be desirable to have an RH level of the air which is selected to provide, for instance, a comfortable indoor climate, for other cleaning functions such as inactivation of microorganisms to function optimally etc. The temperature of the catalyst, $T_{cat}$, is then often be adjusted to an acceptable combination with this given RH level and result in optimal degradation of organic substances.

Fig. 4 illustrates schematically a device consisting of a sensor 3 for measuring the relative humidity of the air, $RH_{air}$; a photocatalytic surface 5 over which the air flows; a light source 6 for irradiating the photocatalytic surface with activating light, and an adjusting device 7 for setting the temperature of the photocatalytic surface, $T_{cat}$; and a control unit 9 for controlling the temperature of the photocatalytic surface, $T_{cat}$, to be within predetermined acceptable combinations with $RH_{air}$ to establish and maintain 0.2-8 monolayers of water molecules on the photocatalytic surface. A temperature sensor 8 senses the temperature of the photocatalytic surface and supplies information to the control unit 9. The control unit also receives information about the current $RH_{air}$ from the humidity sensor 3 and calculates by means of input data a suitable $T_{cat}$ for the catalyst to work optimally and controls the adjusting device 7 for setting this $T_{cat}$. In this case, the photocatalytic surface 5 is arranged on the outside of the light source 6 as a thin film. The surface 5 is heated by the light source 6 and $T_{cat}$ is regulated by a power regulator for the light source or electric supplementary heating of the casing of the light source. In this case the adjusting device 7 thus consists of the light source 6 with the associated power regulator or supplementary heater, which are controlled by the control unit 9. The thickness of the catalytic film must be thinner than the penetration depth of the activating light emitted from the light source. For $TiO_2$,

the thickness must be less than 2 micrometer and preferably less than 1 micrometer. The light source can also be an optical waveguide which transmits light at a wavelength exceeding the optical bandgap energy of the photocatalyst.

A device of this type can be used, for instance, in a room for cleaning of indoor air, the air flowing over the photocatalytic surface by convection. The device requires that a suitable $T_{cat}$ can be achieved on the surface of the light source in order to match the RH of the room air. For indoor air with an RH of 30-70%, this is possible in most cases. The RH of the room air may also be adjusted to a suitable level with a separate RH adjusting system, which means that $T_{cat}$ need only be adjusted in a limited range.

Fig. 5 illustrates an example of a device suitable for integration into an air-conveying system, for instance a circulation system. The device comprises an inlet 1 and an outlet 2 for air that is to be cleaned of organic substances. A humidity sensor 3 measures the relative humidity of the inlet air, $RH_{air}$. Then air flows through the catalytic reactor, which in the embodiment illustrated consists of tubes 4, whose inside is coated with a photocatalytic surface 5, for instance in the form of $TiO_2$ coating. A light source 6, which activates the photocatalytic surface, for instance a UV lamp, is arranged centrally in the tube 4. The air flows in the gap between the light source 6 and the photocatalytic surface 5 and is deflected from one tube to the next so as to pass them in series. For reasons of clarity, the Figure shows only three tubes, but the photocatalytic reactor can have a large number of tubes connected in series and many series of tubes acting in parallel. Other ways of arranging the photocatalytic surface are known in the literature, for instance, in honeycomb structure, membranes etc, and can also be applied in the invention, as can also the above-described variant with a photocatalytic coating on the light source. The efficiency of the photocatalyst is typically in the order of 1%, and therefore a long dwell time over the photocatalyst and a large contact surface between air and photocatalyst are desired. The device further comprises an adjusting device 7 for setting the temperature of the photocatalytic surface, $T_{cat}$, and a temperature sensor 8 for measuring the temperature of the photocatalytic surface. The temperature sensor may also constitute part of the adjusting device, which is illustrated by a dashed line in the Figure. Then the adjusting device has a thermostat function and sets the temperature of the photocatalytic surface, $T_{cat}$, to a reference value from the control unit 9. The control unit 9 receives information about $RH_{air}$ from the sensor 3 and information about $T_{cat}$ from the temperature sensor 8 and regulates by means of the adjusting device 7 the temperature of the photocatalytic surface, $T_{cat}$, to be within predetermined acceptable combinations with $RH_{air}$ to establish and maintain 0.2-8 monolayers of water molecules on the photocatalytic surface.

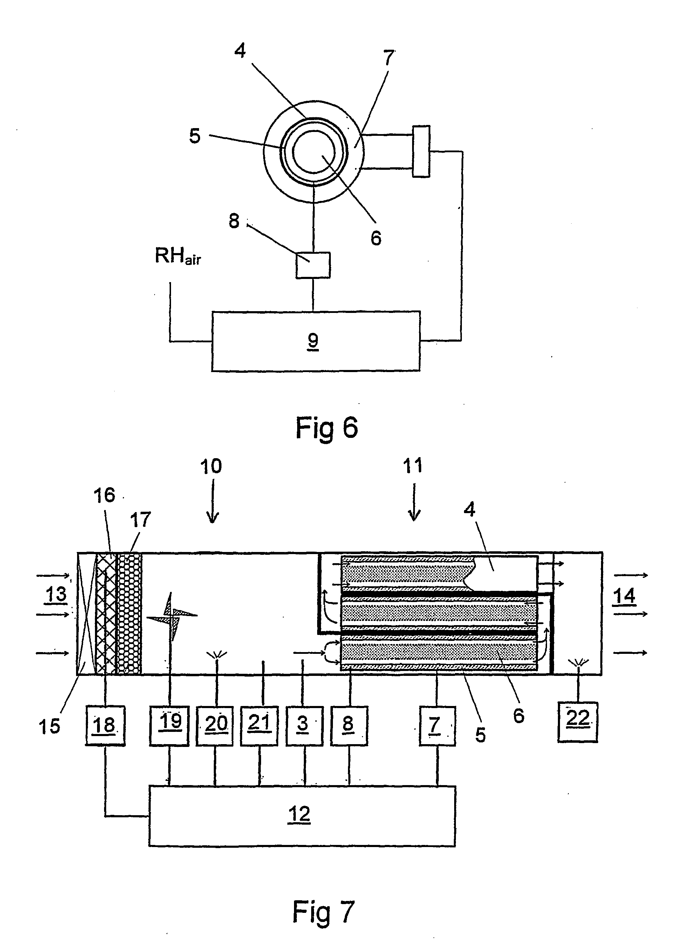

Fig. 6 illustrates on a larger scale a section through a tube 4 with a photocatalytic surface 5, a central light source 6, an adjusting device 7, a temperature sensor 8 and a control unit 9. The adjusting device 7 can be an electric heater which encloses the tube 4, or a system which circulates a tempered liquid through an outer casing surrounding the tube 4.

The embodiment described above can be provided with additional equipment, such as particle filter, adsorption filter and flow control in the same way as the embodiment according to Fig. 7 that will be described below.

Fig. 7 illustrates a device where integrated regulation of $RH_{air}$ and $T_{cat}$ is applied. The device comprises an air-conditioning unit 10, in which the relative humidity of the air, $RH_{air}$, and the flow thereof are regulated; a reactor 11, comprising a photocatalytic surface 5 over which the conditioned air from the air-conditioning unit 10 flows, a light source 6 for irradiating the photocatalytic surface with activating light and an adjusting device 7 for setting the temperature of the photocatalytic surface, $T_{cat}$; and a control unit 12 for integrated control of the air-conditioning unit 10 and the photocatalytic reactor 11 for regulating $RH_{air}$ and $T_{cat}$ so that the combination of $RH_{air}$ and $T_{cat}$ is caused to fall within predetermined acceptable combinations of $RH_{air}$ and $T_{cat}$ to establish and maintain 0.2-8 monolayers of water molecules on the photocatalytic surface. The reactor 11 corresponds to the previously described device according to Figs 5-6 except that control of $T_{cat}$ is now coordinated with control of $RH_{air}$.

Air that is to be cleaned of organic substances enters the air-conditioning unit 10 through an inlet 13, passes through the reactor 11 and leaves the device through an outlet 14. The air may first pass a mechanical filter 15 where dust and other solid particles are separated and after that the air can, if required, be deflected via an adsorption filter 16 and, if required, also via an air-dehumidifying filter 17 which may consist of a hygroscopic material, such as silica gel, activated clay and aluminium silicate. The adsorption filter 16, which suitably is made of active charcoal (AC), has the function of dampening, by physical adsorption, transient fluctuations in the air flow of large amounts of chemical compounds and biological contaminants. By adjusting the dimensions of the AC filter, an appropriate flow of air can be achieved without additional mechanical fans and other costly installations. The charcoal filter can be smaller than in normal charcoal filtering owing to the purifying effect of the subsequent photocatalytic filter. In this way a lower pressure drop can be obtained. In addition, the AC filter need not be regenerated since its optimised function can be obtained by controlled thermal desorption of the AC filter where the accumulated amount of contaminants in a suitable amount is made to flow over the photocatalytic surface and thus is degraded into $CO_2$, $H_2O$ and optionally inorganic anions (mineral acids). Desorption can be effected in periods when the concentration of organic substances in the incoming air is low. The thermal desorption can be performed using a heating device 18, which can also be controlled by the control unit 12. Sensors (not shown) which sense the concentration of organic substances in the air before and after the adsorption filter can supply information to the control unit 12 for controlling the desorption and the connection of the filter in the airflow.

The air-conditioning unit 10 further comprises a controllable fan 19 for controlling the airflow through the device; a humidifying device 20, which can be of a conventional type, for instance mist spray, a flow sensor 21 and an RH sensor 3. The control unit 12 receives information from the sensors 21, 3, 8 and controls the fan 19, the humidifying device 20 and the adjusting device 7 so that a suitable combination of $RH_{air}$ and $T_{cat}$ is achieved. The simultaneous control of the airflow with respect to dwell time and flow over the catalytic surface makes it possible to further optimise the function of the photocatalyst. Also the connection of the air-dehumidifying filter 17 can be controlled by the control unit 12 when incoming air has too high an RH to be matched with a suitable $T_{cat}$ for optimal oxidation. If the air has been dried, also rehumidification of the air before it leaves the device can be desirable. This can be performed using a humidifying device 22 after the reactor 11.

**Claims**

1. A method for photocatalytic oxidation of organic substances in air on a photocatalytic surface of metal oxide, air containing the organic substances being caused to flow over the photocatalytic surface and the surface being irradiated with activating light, **characterised in that** the metal oxide is $TiO_2$ and that the relative humidity of the air ($RH_{air}$), and the temperature of the photocatalytic surface $T_{cat}$ are in such a way as to establish and maintain 0.2-8 monolayers of water molecules on the photocatalytic surface, said correlation being obtained by means of:

   a) measuring $RH_{air}$ and calculating $k_a = \dfrac{P_{H2O}}{\sqrt{2\pi MRT}}$ , where R is the general gas constant, M is the molar mass of $H_2O$, and T is 298 K

   b) using regulated $K_a$ to regulate the temperature of $T_{cat}$ in such a way that

   $$0.2 \times 1.15\times 10^{19} \leq \frac{k_a}{v_0}\exp\left(\frac{E_d}{R \cdot T_{cat}}\right) \leq 8 \times 1.15 \times 10^{19},$$ where $E_d$ is the desorption energy of $H_2O$ molecules from the catalyst surface, $v_0$ is a pre-exponential factor with value $10^{13}$ s$^{-1}$, $1.15 \times 10^{19}$ is the value of a single monolayer of water regulated

2. A method according to claim 1 **characterised in that** the relative humidity of air $RH_{air}$ is lying between 1 % and 60 % when the temperature of the photocatalytic surface $T_{cat}$ is 360 K.

3. A method according to claim 1 **characterised in that** the relative humidity of air $RH_{air}$ is lying between 0.05 % and 4 % when the temperature of the photocatalytic surface $T_{cat}$, is 298 K.

4. A method as in any of the claims 1-3, **characterised in that** the activating light has an energy that exceeds the optical bandgap energy for the photocatalyst.

5. A method as claimed in any of the claims 1-4, **characterised in that** the photocatalytic surface has an increased temperature relative to the ambient air.

6. A method as claimed in any of the claims 1-5, **characterised in that** the air is filtered through a particle filter before it is caused to flow over the photocatalytic surface.

7. A method as claimed in any of the claims 1-6, **characterised in that** the air is dried before it is caused to flow over the photocatalytic surface.

8. A method as claimed in any of the claims 1-7 **characterised in that** part of the organic substances in the air is adsorbed in an adsorption filter before the air is caused to flow over the photocatalytic surface.

9. A method as claimed in claim 8 **characterised in that** the adsorbed organic substances are desorbed from the adsorption filter and caused to flow over the photocatalytic surface.

10. A method as claimed in any of the claims 1-3, **characterised in that** the photocatalytic surface is heated by being arranged on a light-absorbing layer, which in turn is heated by being exposed to light that is absorbed in the layer.

**Patentansprüche**

1. Verfahren zur photokatalytischen Oxidation von organischen Substanzen in Luft auf einer photokatalytischen Oberfläche aus Metalloxid, wobei die die organischen Substanzen beinhaltende Luft zur Strömung über die photokatalytische Oberfläche veranlasst wird und die Oberfläche mit Aktivierungslicht bestrahlt wird, **dadurch gekennzeichnet, dass** das Metalloxid $TiO_2$ ist und dass die relative Luftfeuchtigkeit ($RH_{air}$) und die Temperatur der photokatalytischen Oberfläche $T_{cat}$ so reguliert werden, dass sich 0,2 bis 8 Monoschichten von Wassermolekülen auf der photokatalytischen Oberfläche ansammeln und erhalten bleiben, wobei besagte Korrelation erzielt wird durch:

a) Messen von $RH_{air}$ und Berechnen von $K_a = \dfrac{P_{H2O}}{\sqrt{2\pi MRT}}$, wobei R die allgemeine Gaskonstante, M die Molmasse von $H_2O$, und T 298 K beschreibt

b) Verwendung von $k_a$ zur Regulierung der Temperatur von $T_{cat}$, auf solche Weise, dass 0,2

$$x\ 1{,}15 \times 10^{19} \leq \frac{k_a}{\nu_0} \exp\left(\frac{E_d}{R \cdot T_{cat}}\right) \leq 8 \times 1{,}15 \times 10^{19},$$

wobei $E_d$ die Desorptionsenergie von $H_2O$-Molekülen aus der katalytischen Oberfläche ist, $\nu_0$ ein präexponentieller Faktor mit dem Wert $10^{13}$ $s^{-1}$, und $1{,}15 \times 10^{19}$ der Wert einer einzelnen Monoschicht von Wasser.

2. Verfahren nach Anspruch 1, **dadurch gekennzeichnet, dass** die relative Luftfeuchtigkeit $RH_{air}$ zwischen 1 % und 60 % beträgt, wenn die Temperatur der photokatalytischen Oberfläche $T_{cat}$ 360 K beträgt.

3. Verfahren nach Anspruch 1, **dadurch gekennzeichnet, dass** die relative Luftfeuchtigkeit $RH_{air}$ zwischen 0,05 % und 4 % beträgt, wenn die Temperatur der photokatalytischen Oberfläche $T_{cat}$ 298 K beträgt.

4. Verfahren nach einem der Ansprüche 1 bis 3, **dadurch gekennzeichnet, dass** das Aktivierungslicht eine Energie aufweist, die die optische Bandlückenenergie des Photokatalysators übersteigt.

5. Verfahren nach einem der Ansprüche 1 bis 4, **dadurch gekennzeichnet, dass** die photokatalytische Oberfläche eine höhere Temperatur relativ zur Umgebungsluft aufweist.

6. Verfahren nach einem der Ansprüche 1 bis 5, **dadurch gekennzeichnet, dass** die Luft mit Hilfe eines Partikelfilters gefiltert wird, bevor sie zur Strömung über die photokatalytische Oberfläche veranlasst wird.

7. Verfahren nach einem der Ansprüche 1 bis 6, **dadurch gekennzeichnet, dass** die Luft getrocknet wird, bevor sie zur Strömung über die phocokatalytische Oberfläche veranlasst wird.

8. Verfahren nach einem der Ansprüche 1 bis 7, **dadurch gekennzeichnet, dass** ein Teil der organischen Substanzen in der Luft in einem Adsorptionsfilter adsorbiert wird, bevor die Luft zur Strömung über die photokatalytische Oberfläche veranlasst wird.

9. Verfahren nach Anspruch 8, **dadurch gekennzeichnet, dass** die adsorbierten organischen Substanzen von dem Adsorptionsfilter desorbiert werden und zur Strömung über die photokatalytische Oberfläche veranlasst werden.

10. Verfahren nach einem der Ansprüche 1 bis 3, **dadurch gekennzeichnet, dass** die photokatalytische Oberfläche durch Anordnung auf einer lichtabsorbierenden Schicht erwärmt wird, welche Schicht wiederum erwärmt wird, indem sie Licht ausgesetzt ist, das in der Schicht absorbiert ist.

**Revendications**

1. Procédé d'oxydation photocatalytique de substances organiques présentes dans l'air sur une surface photocatalytique d'un oxyde métallique, l'air contenant les substances organiques étant mis en circulation sur la surface photocatalytique, et cette surface étant irradiée avec de la lumière d'activation,

**caractérisé en ce que**
l'oxyde métallique est $TiO_2$ et l'humidité relative de l'air ($RH_{air}$), et la température de la surface photocatalytique $T_{cat}$ sont régulées de façon à établir et maintenir 0,2-8 monocouches de molécules d'eau sur la surface photocatalytique, cette régulation étant obtenue en :

a) mesurant $RH_{air}$ et calculant $k_a = \dfrac{P_{H2O}}{\sqrt{2\pi MRT}}$, R étant la constante universelle des gaz parfaits, M la masse molaire de $H_2O$ et T 298 K,

b) utilisant $k_a$ pour réguler la température $T_{cat}$ de sorte que $0,2 \times 1,15 \times 10^{19} \leq \dfrac{k_a}{v_0} exp(\dfrac{E_d}{R.T_{cat}}) \leq 8 \times 1,15 \times 10^{19}$, $E_d$ étant l'énergie de désorption de molécules de $H_2O$ de la surface catalytique, $v_0$ étant un facteur pré-exponentiel de valeur $10^{13}$ $s^{-1}$, et $1,15 \times 10^{19}$ étant la valeur d'une seule monocouche d'eau.

2. Procédé conforme à la revendication 1,
   **caractérisé en ce que**
   l'humidité relative de l'air $RH_{air}$ est située entre 1 % et 60 % lorsque la température de la surface photocatalytique $T_{cat}$ est de 360 K.

3. Procédé conforme à la revendication 1,
   **caractérisé en ce que**
   l'humidité relative de l'air Chair est située entre 0,05 % et 4 % lorsque la température de la surface photocatalytique $T_{cat}$ est de 298 K.

4. Procédé conforme à l'une quelconque des revendications 1 à 3,
   **caractérisé en ce que**
   la lumière d'activation a une énergie supérieure à l'énergie de la bande optique interdite pour le photocatalyseur.

5. Procédé conforme à l'une quelconque des revendications 1 à 4,
   **caractérisé en ce que**
   la surface photocatalytique a une température augmentée par rapport à l'air ambiant.

6. Procédé conforme à l'une quelconque des revendications 1 à 5,
   **caractérisé en ce que**
   l'air est filtré au travers d'un filtre à particules avant d'être mis en circulation sur la surface photocatalytique.

7. Procédé conforme à l'une quelconque des revendications 1 à 6,
   **caractérisé en ce que**
   l'air est séché avant d'être mis en circulation sur la surface photocatalytique.

8. Procédé conforme à l'une quelconque des revendications 1 à 7,
   **caractérisé en ce qu'**
   une partie des substances organiques présentes dans l'air est adsorbée dans un filtre d'adsorption avant que l'air mis en circulation sur la surface photocatalytique.

9. Procédé conforme à la revendication 8,
   **caractérisé en ce que**
   les substances organiques adsorbées sont désorbées du filtre d'adsorption et mis en circulation sur la surface photocatalytique.

10. Procédé conforme à l'une quelconque des revendications 1 à 3,
    **caractérisé en ce que**
    la surface photocatalytique est chauffée en étant positionnée sur une couche absorbant de la lumière qui est, à soin tour, chauffée en étant exposée à la lumière qui est adsorbée dans la couche.

Fig 1

Fig 2

Fig 3

Fig 4

Fig 5

Fig 6

Fig 7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0206159 A, Kemitt

- US 200315707 A1, Carmignani

**Non-patent literature cited in the description**

- **Fujishima, A. ; Hashimoto, K ; Watanabe, T.** TiO2 Photocatalysis. Fundamentals and Applications. BKC, Inc, 1999
- **Ollis, D.F. ; Al-Ekabi, H.** Photocatalytic Purification and Treatment of Water and Air. Elsevier, 1993

- **Fox, M.A. ; Dulay, M.T.** *Chem. Rev.,* 1993, vol. 93, 341
- **Mills, A. ; Le Hunte, S.** *J. Photochem. Photobiol. A,* 1997, vol. 108, 1